# DEMANDE DE BREVET EUROPEEN

(11) **EP 0 970 690 A1**
(43) Date de publication de la demande: **12.01.2000**
(21) Numéro de dépôt: 99401437.1
(22) Date de dépôt: 11.06.1999
(51) Int. Cl.: A61K 7/48

(54) **Composition cosmétique et/ou dermatologique contenant des dérivés d'acide salicylique et procédé d'introduction des dits dérivés dans une composition aqueuse**

(30) Priorité: 06.07.1998 FR 9808617
(71) Demandeur: L'OREAL, 75008 Paris (FR)
(72) Inventeur: Simon, Pascal, 94400 Vitry/S/Seine (FR); Bordeaux, Dominique, 91310 Longpont/S/Orge (FR)
(74) Mandataire: Rasson, Catherine

(57) **Abrégé**

La présente invention se rapporte à une composition cosmétique et/ou dermatologique comprenant au moins un dérivé d'acide salicylique de formule (1), notamment un dérivé salifié, et au moins un éther oxyéthyléné oxypropyléné d'alcool gras ayant de 12 à 22 atomes de carbone, et à un procédé d'introduction du dit dérivé d'acide salicylique dans une composition aqueuse à l'aide d'un tel éther.

La présente invention a aussi pour objet l'utilisation de la composition de l'invention en particulier pour le soin et/ou le maquillage de la peau, des muqueuses et/ou des fibres kératiniques, et plus particulièrement pour lutter contre les signes du vieillissement cutané et/ou pour améliorer l'éclat du teint et/ou pour lisser la peau du visage et/ou du corps et/ou pour traiter les rides et les ridules de la peau et/ou pour stimuler le processus de renouvellement épidermique et/ou pour traiter l'acné et/ou les désordres cutanés.

La composition de l'invention peut se présenter sous forme de solutions ou gels aqueux ou sous forme d'émulsions.

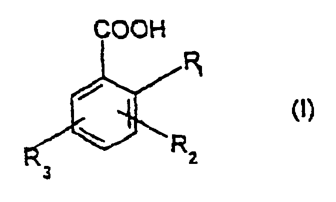

## Description

La présente invention se rapporte à une composition cosmétique et/ou dermatologique comprenant au moins un dérivé d'acide salicylique, notamment un dérivé salifié, et au moins un éther oxyéthyléné oxypropyléné d'alcool gras ayant de 12 à 22 atomes de carbone, et à un procédé d'introduction du dit dérivé d'acide salicylique dans une composition aqueuse à l'aide d'un tel éther.

La présente invention a aussi pour objet l'utilisation de la composition de l'invention en particulier pour le soin et/ou le maquillage de la peau, des muqueuses et/ou des fibres kératiniques, et plus particulièrement pour lutter contre les signes du vieillissement cutané et/ou pour améliorer l'éclat du teint et/ou pour lisser la peau du visage et/ou du corps et/ou pour traiter les rides et les ridules de la peau et/ou pour stimuler le processus de renouvellement épidermique et/ou pour traiter l'acné et/ou les désordres cutanés.

Il est connu d'utiliser des dérivés de l'acide salicylique comme agent kératolytique pour traiter l'acné et comme agent d'antivieillissement dans des compositions cosmétiques et/ou dermatologiques. Ainsi, les documents FR-A-2 581 542 et EP-A-378 936 décrivent de tels dérivés.

Les dérivés d'acide salicylique sont d'un grand intérêt, étant donné leurs effets biologiques sur la peau, notamment sur les principaux signes cliniques du vieillissement cutané que sont les ridules et rides, la désorganisation du "grain" de la peau, la modification du teint de la peau et la perte de fermeté et de tonicité de la peau. En outre, ils présentent l'avantage par rapport à l'acide salicylique, d'avoir une activité kératolytique supérieure et une activité bactériostatique efficace.

Cependant, l'utilisation de ces dérivés pose un problème dans la mesure où ils peuvent provoquer des picotements, des démangeaisons, des tiraillements après leur application, pouvant conduire à un important inconfort. L'utilisation de ces composés pour les utilisateurs à peau sensible est donc souvent rédhibitoire.

Pour surmonter cet inconvénient, la demanderesse a découvert de nouveaux dérivés salifiés divalents de l'acide salicylique permettant de diminuer l'irritation induite par ces acides. Ces nouveaux dérivés font l'objet de la demande FR-9705412 indiquée ici pour référence.

Toutefois, contrairement à l'acide salicylique dont l'introduction dans la phase aqueuse des compositions cosmétiques, dans les conditions normales de pH (4-7) et aux taux habituels d'utilisation, ne pose pas de problème, l'utilisation des dérivés de l'acide salicylique décrits dans les documents FR-A-2 581 542 et EP-A-378 936 aussi bien que celle des dérivés salifiés monovalents ou divalents posent un problème dans la mesure où ces composés se présentent sous forme cristalline et où ils sont difficilement ou pas du tout solubles dans l'eau et dans les huiles traditionnellement utilisées dans les domaines cosmétique et dermatologique.

Ainsi, si on les introduit tels quels dans les compositions cosmétiques et/ou dermatologiques, ils restent à l'état de cristaux, ce qui rend l'utilisation de la composition les contenant, inefficace pour le traitement de la peau.

Par ailleurs, les dérivés salifiés ne sont pas solubles dans les solvants usuels tels que les alcools inférieurs comme l'éthanol ou l'isopropanol, ou dans des solvants tels que l'octyldodécanol, certains glycols, les alcools gras à chaîne courte (< C₁₂) ou les esters à chaîne courte (< C₁₂).

Il subsiste donc le besoin de pouvoir introduire les dérivés de l'acide salicylique et leurs sels dans des compositions cosmétiques et/ou dermatologiques, notamment dans la phase aqueuse de telles compositions.

La demanderesse a trouvé de façon inattendue que les éthers oxyéthylénés oxypropylénés d'alcool gras en C₁₂ à C₂₂ permettent l'introduction des dérivés de l'acide salicylique dans la phase aqueuse des compositions cosmétiques et/ou dermatologiques, sans que se produise une recristallisation de ces dérivés.

On entend par "éthers oxyéthylénés oxypropylénés d'alcool gras en C₁₂ à C₂₂" les éthers d'alcool gras ayant de 12 à 22 atomes de carbone, comportant au moins un groupement oxyéthyléné et au moins un groupement oxypropyléné.

L'invention a donc pour objet une composition cosmétique et/ou dermatologique, caractérisée par le fait qu'elle comprend au moins un dérivé d'acide salicylique de formule (I) ou un sel monovalent ou divalent d'un tel dérivé de formule (I) : 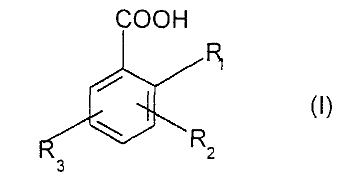 dans laquelle
R₁ représente un groupement hydroxyle ou une fonction ester de formule : 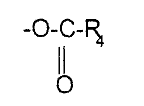 où R₄ est un groupement aliphatique, saturé ou insaturé ayant de 1 à 18 atomes de carbone, ou une fonction amine ou thiol éventuellement substituée par une chaîne alkyle ayant de 1 à 12 atomes de carbone ;
R₂ et R₃ sont, indépendamment l'un de l'autre, en position 3, 4, 5 ou 6 sur le noyau benzénique et représentent l'un ou l'autre mais non simultanément un hydrogène, ou bien représentent, de façon identique ou différente, un groupe 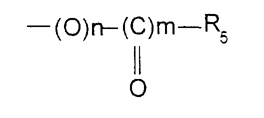
où n et m, indépendamment l'un de l'autre, sont 0 ou 1, et R₅ représente un hydrogène, une chaîne aliphatique saturée ayant de 1 à 18 atomes de carbone, linéaire, ramifiée ou cyclisée, une chaîne insaturée ayant de 3 à 18 atomes de carbone, portant une ou plusieurs doubles liaisons conjuguées ou non, les chaînes pouvant être substituées par au moins un substituant choisi parmi les atomes d'halogène, les groupements trifluorométhyle, les groupements hydroxyle sous forme libre ou estérifiée par un acide ayant de 1 à 6 atomes de carbone, ou bien une fonction carboxyle, libre ou estérifiée par un alcool inférieur ayant de 1 à 6 atomes de carbone, une chaîne aromatique ayant de 6 à 10 atomes de carbone,
et au moins un éther d'alcool gras ayant de 12 à 22 atomes de carbone, comprenant au moins un groupement oxyéthyléné et au moins un groupement oxypropyléné.

L'ajout de l'éther d'alcool gras au dérivé d'acide salicylique permet d'introduire le dit dérivé dans une phase aqueuse.

Aussi, l'invention a encore pour objet un procédé pour introduire un dérivé d'acide salicylique de formule (I) ou un sel monovalent ou divalent d'un tel dérivé dans une composition cosmétique et/ou dermatologique contenant de l'eau, consistant à ajouter au dit dérivé au moins un éther d'alcool gras ayant de 12 à 22 atomes de carbone, comprenant au moins un groupement oxyéthyléné et au moins un groupement oxypropyléné.

Dans la formule (I), la chaîne alkyle de la fonction amine ou thiol peut être linéaire, ramifiée saturée ou non et éventuellement substituée par au moins un hydroxyle. De même, la chaîne aromatique comprend une fonction aromatique liée à une chaîne alkyle en C₁ à C₄ linéaire ou ramifiée saturée ou non et éventuellement substituée par au moins un hydroxyle.

De préférence, R₂ représente l'hydrogène et le dérivé de formule (I) utilisé dans la composition selon l'invention est donc un dérivé de formule (II) : 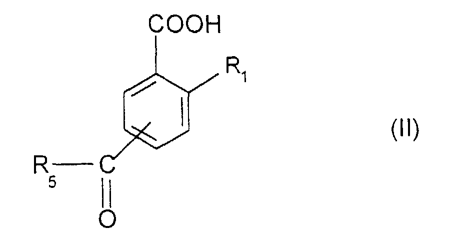 dans laquelle R₁ et R₅ ont la signification indiquée ci-dessus.

De préférence, le radical R₅ est en position 5 du noyau benzénique et comporte au moins 3 atomes de carbone. Par exemple, R₅ représente une chaîne aliphatique saturée ayant de 3 à 15 atomes de carbone, et mieux de 7 à 11 atomes de carbone.

Selon un mode préféré de réalisation de l'invention, R₁ représente un groupement hydroxyle.

Les sels des dérivés de formule (I) peuvent être des sels monovalents de formule (III) :

AR⁻S⁺ (III)

ou des sels divalents de formule (IV) :

AR⁻ S²⁺ AR⁻ (IV)

dans lesquelles :
S⁺ représente un cation inorganique monovalent ;
S²⁺ représente un cation inorganique divalent ;
et AR⁻ correspond à la formule (V) : 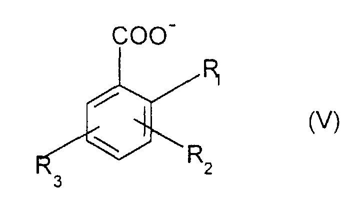 dans laquelle R₁, R₂ et R₃ ont les significations indiquées ci-dessus.

De façon avantageuse, les dérivés de l'acide salicylique sont choisis parmi les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique, les sels monovalents de ces acides, les sels divalents de ces acides et leurs mélanges. On peut aussi utiliser les acides 5-tert-octylsalicylique, 3-tert-butyl-5 ou 6-méthylsalicylique, 3,5-diisopropylsalicylique, 5-butoxysalicylique, 5-octyloxysalicylique, propanoyl-5-salicylique, n-hexadecanoyl-5 salicylique, n-oléoyl 5-salicylique, benzoyl-5 salicylique, leurs sels monovalents et divalents et leurs mélanges.

Les cations inorganiques monovalents sont en particulier ceux des éléments de la colonne lA de la classification périodique. Les cations monovalents préférés sont ceux de sodium et de potassium.

Les cations inorganiques divalents sont en particulier ceux des éléments des colonnes IB, IIA, IIB, IIIB, IIIA, IVB, VB, VIB, VIIB, VIII de la classification périodique. Les cations divalents préférés sont ceux des éléments des colonnes IB, IIA, IIB, VIIB, VIII et encore mieux ceux des éléments des colonnes IIA, IIB et VIIB de la classification périodique et notamment ceux de strontium, de calcium, de magnésium, de baryum et de manganèse.

Les dérivés préférés utilisés dans la composition selon l'invention sont le sel de strontium de l'acide 5-octanoyl-salicylique, le sel de calcium de l'acide 5-octanoylsalicylique, le sel de magnésium de l'acide 5-octanoyl-salicylique.

L'éther d'alcool gras ayant de 12 à 22 atomes de carbone, comprenant au moins un groupement oxyéthyléné et au moins un groupement oxypropyléné, utilisé dans la composition selon l'invention peut comprendre par exemple de 5 à 30 et de préférence de 9 à 25 groupements oxyéthylénés et de 1 à 25 et de préférence de 1 à 7 groupements oxypropylénés.

Comme éthers utilisables dans l'émulsion selon l'invention, on peut citer par exemple l'ester acétique d'éther isocétylique oxyéthyléné oxypropyléné comportant en moyenne 2 groupements oxypropylénés et 20 groupements oxyéthylénés (nom CTFA : PPG-2 isoceteth-20 acetate), tel que le produit commercialisé sous la dénomination CUPL PIC^{R} par la société BERNEL, l'éther oxyéthyléné oxypropyléné d'alcool cétylique comportant en moyenne 5 groupements oxypropylénés et 20 groupements oxyéthylénés (nom CTFA : PPG-5 ceteth-20), tel que le produit commercialisé sous la dénomination Procetyl AWS^{R} par la société CRODA, et l'éther oxyéthyléné oxypropyléné de lauryl glycol comportant en moyenne 1 groupement oxypropyléné et 9 groupements oxyéthylénés (nom CTFA : PPG-1 PEG-9 lauryl glycol ether), tel que le produit commercialisé sous la dénomination Eumulgin L^{R} par la société HENKEL. On peut utiliser aussi un mélange de plusieurs éthers d'alcools gras.

Ces composés ont l'avantage d'éviter la recristallisation des dérivés d'acide salicylique de formule (I) et de leurs sels et de permettre leur incorporation dans la phase aqueuse des compositions cosmétiques et/ou dermatologiques.

Le dérivé de formule (I) ou le sel d'un tel dérivé est de préférence présent en une quantité efficace pour assurer le résultat escompté. Il peut être par exemple présent dans l'émulsion selon l'invention en une quantité allant de 0,001 à 20 %, de préférence de 0,01 à 10 % et mieux de 0,1 à 5 % en poids du poids total de la composition.

L'éther d'alcool gras utilisé dans l'émulsion selon l'invention doit être présent en une quantité efficace pour éviter la recristallisation du dérivé d'acide salicylique. Il est de préférence présent dans la composition selon l'invention en une quantité allant de 0,1 à 30 % et de préférence de 1 à 10 % en poids du poids total de la composition.

Le rapport pondéral entre le dérivé de formule (I) ou le sel d'un tel dérivé et l'éther d'alcool gras va de préférence de 2/10 à 0,01/10 et mieux de 1/10 à 0,01/10.

Le mélange de l'éther d'alcool gras et du dérivé d'acide salicylique peut être introduit sans difficulté dans une phase aqueuse.

Cette phase aqueuse peut contenir en plus notamment un ou plusieurs alcools et/ou polyols tels que l'éthanol, la glycérine, le butylèneglycol, l'isoprèneglycol, le propylèneglycol, le sorbitol, dans des concentrations allant préférentiellement de 1 à 20 % en poids du poids total de la composition.

La composition de l'invention peut se présenter sous toute forme galénique appropriée pour une application topique et notamment sous forme d'une solution aqueuse, d'un gel aqueux, d'une dispersion, d'une émulsion huile dans eau (H/E) ou eau dans huile (E/H), d'une émulsion triple (E/H/E ou H/E/H). Elles peuvent également se trouver sous une forme vectorisée, comme par exemple sous forme de nanocapsules, de liposomes, de nanoémulsions, d'oléosomes.

Selon un mode préféré de réalisation de l'invention, la composition est une émulsion H/E.

Ces compositions sont destinées à une application topique et comprennent de manière appropriée un milieu physiologiquement acceptable. Par milieu physiologiquement acceptable, on entend un milieu compatible avec la peau, les muqueuses (y compris l'intérieur des paupières et les lèvres), les ongles et/ou les fibres kératiniques (cheveux et cils).

Lorsque la composition est une émulsion, cette dernière contient de façon classique au moins une huile et éventuellement un émulsionnant approprié.

La nature de la phase huileuse rentrant dans la composition des émulsions n'est pas critique et elle peut ainsi être constituée par tous les corps gras et notamment les huiles, classiquement utilisés dans les domaines cosmétique et dermatologique.

Parmi les huiles utilisables dans l'émulsion de l'invention, on peut notamment citer par exemple les huiles végétales (jojoba, avocat, sésame, tournesol, maïs, soja, carthame, pépins de raisin), les huiles minérales (vaseline, isoparaffines, dioctylcyclohexane), les huiles de synthèse (myristate d'isopropyle, octanoate de cétéaryle, polyisobutylène, palmitate d'éthyl-hexyle, alkyl benzoates), les huiles de silicone (cyclométhicone telle que le cyclohexadimethylsiloxane, polydiméthylsiloxanes, polyméthylphénylsiloxanes, polydiméthylfluorosiloxanes) et les huiles fluorées. Les autres corps gras susceptibles d'être présents dans la phase huileuse peuvent être par exemple les acides gras, les alcools gras et les cires.

La phase huileuse de l'émulsion peut représenter de 1 à 50 % et mieux de 5 à 40 % en poids du poids total de l'émulsion.

Les émulsions peuvent contenir au moins un agent émulsionnant permettant de stabiliser l'interface huile/eau. L'agent émulsionnant peut être choisi parmi les agents émulsionnants amphotères, anioniques, cationiques ou non-ioniques, utilisés seuls ou en mélange. Ces agents émulsionnants sont choisis de manière appropriée suivant l'émulsion à obtenir (E/H ou H/E).

Pour les émulsions H/E, on peut citer par exemple les agents émulsionnants suivants :
- comme agents émulsionnants amphotères, les N-acyl-aminoacides tels que les N-alkyl-aminoacétates et le cocoamphodiacetate disodique et les oxydes d'amines tels que l'oxyde de stéaramine ;
- comme agents émulsionnants anioniques, les acylglutamates tels que le "disodium hydrogenated tallow glutamate" (Amisoft HS-21^{R} commercialisé par la société AJINOMOTO) ; les acides carboxyliques et leurs sels tels que le stéarate de sodium ; les esters phophoriques et leurs sels tels que le "DEA oleth-10 phosphate" ; les sulfosuccinates tels que le "Disodium PEG-5 citrate lauryl sulfosuccinate" et le "Disodium ricinoleamido MEA sulfosuccinate" ;
- comme agents émulsionnants cationiques, les alkyl imidazolidinium tels que l'éthosulfate d'isostéaryl-éthylimidazolidinium comme le Monaquat ISIES^{R} commercialisé par la société MONA ; les sels d'ammonium tels que le chlorure de N,N,N-triméthyl-1-docosanaminium (Behentrimonium chloride) ;
- comme agents émulsionnants non-ioniques, les alcools gras tels que l'alcool cétylique ; les phosphates tels que le "Triceteareth-4 phosphate" ; les esters et éthers d'oses tels que le "Methylglucose sesquistearate", le "PEG-20 methylglucose sesquistearate", le "Sucrose stearate", le "Sorbitan stearate", le "Sucrose cocoate" et le "Polyglyceryl-3 methyl glucose distearate" ainsi que le mélange de stéarate de sorbitan et de cocoate de sucrose commercialisé par la société ICI sous la dénomination d'Arlatone 2121^{R} ; les esters de polyol, notamment de glycérol ou de sorbitol, tels que le stéarate de glycéryle, le stéarate de polyglycéryl-2, le stéarate de sorbitan ; les éthers de glycérol tels que le "Polyglyceryl-3 decyl tetradecyl ether" ; les esters oxyéthylénés tels que le "PEG-30 glyceryl stearate", le "Polysorbate 60", le "PEG-50 stearate" le "PEG-25 propylene glycol stearate", le "PEG-30 glyceryl cocoate" et le "PPG-2 isoceteth-20 acetate" ; les éthers oxyéthylénés tels que "Octyldodeceth-25", le "PEG-22 dodecyl glycol copolymer", le "Steareth-20", le "Dihydrocholeth-30", le "Beheneth-10", le "PEG-5 soya sterol" ; les éthers d'oses tels que le "Caprylyl/capryl glucoside" .

Pour les émulsions E/H, on peut citer par exemple comme agents émulsionnants les esters gras de polyol, notamment de glycérol ou de sorbitol, et notamment les esters isostéariques, oléiques et ricinoléiques de polyol tels que le mélange de petrolatum, d'oléate de polyglycéryl-3, d'isostéarate de glycéryle, d'huile de ricin hydrogénée et d'ozokérite, vendu sous la dénomination Protegin W^{R} par la société GOLDSCHMIDT, l'isostéarate de sorbitan, le di-isostéarate de polyglycéryle, le sesquiisostéarate de polyglycéryle-2 ; les esters et éthers d'oses tels que le "Methyl glucose dioleate" ; les esters gras tels que le lanolate de magnésium ; les dimethicone copolyols tels que le "Laurylmethicone copolyol" vendu sous la dénomination "Dow Corning 5200 Formulation Aid" par la société DOW CORNING et le "Cetyl dimethicone copolyol" vendu sous la dénomination Abil EM 90^{R} par la société GOLDSCHMIDT.

En outre, de façon connue, les compositions de l'invention peuvent contenir des adjuvants habituels dans les domaines cosmétique et dermatologique, tels que les actifs hydrophiles ou lipophiles, les conservateurs, les antioxydants, les parfums, les charges, les matières colorantes (pigments ou colorants), les filtres solaires, et encore les vésicules lipidiques. Ces adjuvants sont utilisés dans les proportions habituelles dans le domaine cosmétique ou dermatologique, et par exemple de 0,01 à 20 % du poids total de l'émulsion, et ils sont, selon leur nature, introduits dans la phase aqueuse ou dans la phase huileuse de la composition, ou encore dans des vésicules.

Selon la fluidité de la composition que l'on souhaite obtenir, on peut y ajouter un ou plusieurs gélifiants comme les argiles, les gommes polysaccharides et leurs dérivés (gomme de xanthane, carboxyméthyl-hydroxypropyl guar), les polymères carboxyvinyliques ou carbomers. Ces gélifiants sont généralement utilisés à des concentrations allant de 0,1 à 10 %, de préférence de 0,1 à 5 % et mieux de 0,1 à 3 % du poids total de la composition.

Les quantités des différents constituants des compositions selon l'invention sont celles classiquement utilisées dans les domaines considérés. La nature des adjuvants et leurs quantités doivent être telles qu'elles ne modifient pas les propriétés des compositions selon l'invention.

Les compositions, objet de l'invention, trouvent leur application dans un grand nombre de traitements cosmétiques et/ou dermatologiques de la peau, des muqueuses et/ou des cheveux, y compris le cuir chevelu, notamment pour la protection, le soin, le nettoyage et/ou le maquillage de la peau et/ou des muqueuses, pour la protection et/ou le soin des cheveux et/ou pour le traitement thérapeutique de la peau, des cheveux et/ou des muqueuses et plus spécialement des lèvres.

Les compositions selon l'invention peuvent par exemple être utilisées dans des produits de soin ou de nettoyage pour le visage sous forme de lotions, crèmes ou laits, comme produits de maquillage (peau et lèvres) par incorporation de charges, de pigments ou de colorants, ou comme composition solaire par incorporation de filtre(s) solaire(s).

Les compositions de l'invention se sont révélées particulièrement appropriées pour le soin et/ou le maquillage de la peau et/ou des muqueuses et/ou des fibres kératiniques d'être humain, et notamment pour lutter contre les signes du vieillissement cutané et/ou pour lisser la peau du visage et/ou du corps et/ou pour traiter les rides et les ridules de la peau et/ou pour stimuler le processus de renouvellement épidermique et/ou pour traiter l'acné et/ou pour traiter les désordres cutanés.

Par désordres cutanés, on entend en particulier le zona, les brûlures, l'eczéma, la démodécie, l'ulcère cutané, la fibrose, le contrôle des cicatrisations, le psoriasis, les prurits, les dermatites, l'ichtyose, les cors et les verrues.

Aussi, l'invention a encore pour objet un procédé de traitement cosmétique pour la protection, le soin, le nettoyage et/ou le maquillage de la peau et/ou des muqueuses et/ou des fibres kératiniques, consistant à appliquer sur la peau et/ou les muqueuses et/ou les fibres kératiniques, une composition telle que définie ci-dessus.

L'invention a encore pour objet un procédé de traitement cosmétique pour lutter contre les signes du vieillissement cutané et/ou améliorer l'éclat du teint et/ou lisser la peau du visage et/ou du corps et/ou traiter les rides et les ridules de la peau et/ou stimuler le processus de renouvellement épidermique, consistant à appliquer sur la peau une composition telle que définie ci-dessus.

L'invention a aussi pour objet l'utilisation de la composition définie ci-dessus pour la fabrication d'une composition dermatologique destinée à lutter contre les signes du vieillissement cutané et/ou à lutter contre l'acné et/ou à lutter contre les désordres cutanés.

D'autres caractéristiques et avantages de l'invention ressortiront mieux des exemples qui suivent, donnés à titre illustratif et non limitatif.

Les exemples suivants 1 et 2 illustrent le procédé de préparation de dérivés salifiés pouvant être utilisés dans la composition selon l'invention.

### Exemple 1 : Préparation du 5-octanoyl-salicylate de strontium

Dans un ballon de 500 ml muni d'un agitateur, d'un thermomètre et d'un réfrigérant ascendant, on introduit 39,6 g d'acide 5-octanoyl-salicylique (0,15 mole) dans 250 ml d'isopropanol. Quand la dissolution est totale, on introduit par petites fractions, 7,5 g de carbonate de strontium (0,075 mole). Le milieu devient épais et laiteux ; on ajoute alors 50 ml d'eau et on tiédit à 40-45°C.

Dès que tout le carbonate de strontium est introduit, le milieu devient progressivement limpide. On concentre alors, à sec, à l'évaporateur rotatif. On obtient 43,9 g d'une poudre blanche.

### Analyse élémentaire : conforme.

### Exemple 2 : Préparation du 5-octanoyl-salicylate de calcium

Dans un ballon de 500 ml, on introduit 39,6 g d'acide 5-octanoyl-salicylique (0,15 mole) dans 300 ml d'isopropanol et 150 ml d'eau. Puis on ajoute par petites fractions, 7,5 g de carbonate de calcium (0,075 mole). On laisse sous agitation à température ambiante pendant 3 heures, puis on concentre à sec sous vide à l'évaporateur rotatif.

On obtient 42,1 g d'une poudre blanche.

### Analyse élémentaire : conforme.

### Exemple de formulation 1 : Crème H/E de soin quotidien pour le lissage du teint

| *Phase A* | |
|---|---|
| Huile d'amande d'abricot | 22 % |
| Alcool béhénylique | 1,2 % |
| Monostéarate de sorbitan | 2,55 % |
| | |

| *Phase B* | |
|---|---|
| Polymère carboxyvinylique | 0,4 % |
| Gomme de xanthane | 0,15 % |
| Huile de Purcellin liquide ( mélange de myristate | |
| d'isopropyle et octanoate de cétéaryle) | 3 % |
| | |

| *Phase C* | |
|---|---|
| Monostéarate de sorbitan oxyéthyléné OE-20 | 1,85 % |
| Glycérine | 3 % |
| Triéthanolamine | 0,4 % |
| PPG-2 isoceteth-20 acetate | 10 % |
| 5-octanoyl-salicylate de strontium | 2 % |
| conservateurs | 0,3 % |
| Parfum | 0,2 % |
| Eau | qsp 100 % |

Les phases A et B sont chauffées à 70-75°C et mélangées. La phase C est portée à 70°C, et homogénéisée dans le mélange de A et B à la turbine Moritz de type rotor stator. On obtient une crème blanche brillante lisse.

### Exemple de formulation 2 : Crème H/E de soin quotidien pour le lissage du teint

Une seconde composition est réalisée en remplaçant dans l'exemple de formulation 1, le 5-octanoyl-salicylate de strontium par le 5-octanoyl-salicylate de calcium.

On obtient une crème blanche brillante lisse.

### Exemple de formulation 3 : Crème H/E de soin quotidien pour le lissage du teint

Une autre composition est réalisée en remplaçant dans l'exemple de formulation 1, le "PPG-2 isoceteth-20 acetate" par le "PPG-1 PEG-9 lauryl glycol ether".

On obtient une crème blanche brillante lisse.

### Exemple de formulation 4 : Crème H/E de soin quotidien, anti-rides pour peaux sensibles

| *Phase A* | |
|---|---|
| Polymère carboxyvinylique | 0,3 % |
| (Acrylates / C₁₀-C₃₀ alkyl acrylate crosspolymer) | |
| Benzoate d'alcools C₁₂-C₁₅ | 7 % |
| Cyclohexadimethyisiloxane | 5 % |
| | |

| *Phase B* | |
|---|---|
| glycérine | 7 % |
| PPG-2 isoceteth-20 acetate | 10 % |
| 5-octanoyl-salicylate de calcium | 2 % |
| conservateurs | 0,3 % |
| Parfum | 0,2 % |
| Eau | qsp 100 % |

La phase B est chauffée à 70-75°C jusqu'à solubilisation totale. La phase A est portée à 70°C et homogénéisée dans la phase B à la turbine Moritz de type rotor stator. On obtient une crème blanche brillante lisse d'aspect gélifiée.

### Exemple de formulation 5 : Crème H/E de soin quotidien, anti-rides pour peaux sensibles

Une autre composition est réalisée en remplaçant dans l'exemple de formulation 3, le 5-octanoyl-salicylate de calcium par le 5-octanoyl-salicylate de strontium.

On obtient une crème blanche brillante lisse.

### Exemple de formulation 6 : Crème H/E de soin quotidien, anti-rides pour peaux sensibles

Une autre composition est réalisée en remplaçant dans l'exemple de formulation 1, le "PPG-2 isoceteth-20 acetate" par le "PPG-1 PEG-9 lauryl glycol ether".

On obtient une crème blanche brillante lisse.

### Exemple de formulation 7 : Gel transparent de soin quotidien pour le lissage du teint

| | |
|---|---|
| Carboxyméthyl-hydroxypropyl guar | 0,6 % |
| Gomme de xanthane | 0,25 % |
| Polysorbate 20 | 0,2 % |
| Butylène glycol | 3 % |
| Acide 5-octanoyl-salicylique | 2 % |
| PPG-2 isoceteth-20 acetate | 12 % |
| conservateurs | 0,3 % |
| Parfum | 0,2 % |
| Eau | qsp 100 % |

## Revendications

1. Composition cosmétique et/ou dermatologique, caractérisée par le fait qu'elle comprend au moins un dérivé d'acide salicylique de formule (I) ou un sel monovalent ou divalent d'un tel dérivé de formule (I) : 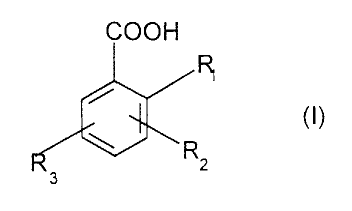 dans laquelle
R₁ représente un groupement hydroxyle ou une fonction ester de formule : 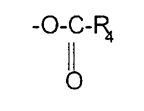 où R₄ est un groupement aliphatique, saturé ou insaturé ayant de 1 à 18 atomes de carbone, ou une fonction amine ou thiol éventuellement substituée par une chaîne alkyle ayant de 1 à 12 atomes de carbone.
R₂ et R₃ sont, indépendamment l'un de l'autre, en position 3, 4, 5 ou 6 sur le noyau benzénique et représentent l'un ou l'autre mais non simultanément un hydrogène, ou bien représentent, de façon identique ou différente, un groupe 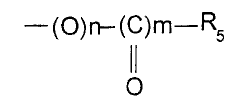 où n et m, indépendamment l'un de l'autre, sont 0 ou 1, et R₅ représente un hydrogène, une chaîne aliphatique saturée ayant de 1 à 18 atomes de carbone, linéaire, ramifiée ou cyclisée, une chaîne insaturée ayant de 3 à 18 atomes de carbone, portant une ou plusieurs doubles liaisons conjuguées ou non, les chaînes pouvant être substituées par au moins un substituant choisi parmi les atomes d'halogène, les groupements trifluorométhyle, les groupements hydroxyle sous forme libre ou estérifiée par un acide ayant de 1 à 6 atomes de carbone, ou bien une fonction carboxyle, libre ou estérifiée par un alcool inférieur ayant de 1 à 6 atomes de carbone, une chaîne aromatique ayant de 6 à 10 atomes de carbone,
et au moins un éther d'alcool gras ayant de 12 à 22 atomes de carbone, comprenant au moins un groupement oxyéthyléné et au moins un groupement oxypropyléné.

2. Composition selon la revendication 1, caractérisée en ce que le radical R₅ représente une chaîne aliphatique saturée ayant de 3 à 15 atomes de carbone.

3. Composition selon la revendication 1 ou 2, caractérisée en ce que R₁ représente un groupement hydroxyle.

4. Composition selon l'une quelconque des revendications précédentes, caractérisée en ce que R₅ est en position 5 du noyau benzénique et R₂ représente l'hydrogène.

5. Composition selon l'une quelconque des revendications précédentes, caractérisée en ce que le dérivé d'acide salicylique de formule (I) est choisi parmi les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique, 5-tert-octylsalicylique, 3-tert-butyl-5- ou 6-méthylsalicylique, 3,5-diisopropylsalicylique, 5-butoxysalicylique, 5-octyloxysalicylique, propanoyl-5-salicylique, n-hexadecanoyl-5-salicylique, n-oléoyl-5-salicylique, benzoyl-5 salicylique, leurs sels monovalents et divalents et leurs mélanges.

6. Composition selon l'une quelconque des revendications précédentes, caractérisée en ce que le sel du dérivé d'acide salicylique de formule (I) est choisi parmi les sels de strontium, de calcium, de magnésium, de baryum et de manganèse.

7. Composition selon l'une quelconque des revendications précédentes, caractérisée en ce que le sel de dérivé d'acide salicylique de formule (I) est choisi parmi le sel de strontium de l'acide 5-octanoyl salicylique, le sel de calcium de l'acide 5-octanoyl salicylique, le sel de magnésium de l'acide 5-octanoyl salicylique et leurs mélanges.

8. Composition selon l'une quelconque des revendications précédentes, caractérisée en ce que le dérivé d'acide salicylique de formule (I) ou le sel dudit dérivé est présent en une quantité allant de 0,001 à 20 % et de préférence de 0,01 à 10 % en poids du poids total de la composition.

9. Composition selon l'une quelconque des revendications précédentes, caractérisée en ce que l'éther d'alcool gras comprend de 5 à 30 groupements oxyéthylénés et de 1 à 25 groupements oxypropylénés.

10. Composition selon l'une quelconque des revendications précédentes, caractérisée en ce que l'éther d'alcool gras est choisi parmi le PPG-2 isoceteth-20 acetate, le PPG-5 ceteth-20, le PPG-1 PEG-9 lauryl glycol ether et leurs mélanges.

11. Composition selon l'une quelconque des revendications précédentes, caractérisée en ce que l'éther d'alcool gras est présent en une quantité allant de 0,1 à 30 % et de préférence de 1 à 10 % en poids du poids total de la composition.

12. Composition selon l'une quelconque des revendications précédentes, caractérisée en ce que le rapport pondéral entre le dérivé de formule (I) ou le sel d'un tel dérivé et l'éther d'alcool gras va de préférence de 2/10 à 0,01/10 et mieux de 1/10 à 0,01/10.

13. Composition selon l'une quelconque des revendications précédentes, caractérisée en ce qu'elle se présente sous forme d'une solution aqueuse, d'un gel aqueux, d'une dispersion, d'une émulsion huile-dans-eau ou eau-dans-huile ou d'une émulsion triple.

14. Composition selon l'une quelconque des revendications précédentes, caractérisée en ce qu'elle comprend en outre au moins un tensioactif.

15. Composition selon l'une quelconque des revendications précédentes, caractérisée en ce qu'elle comprend une phase huileuse représentant de 1 à 50 % et de préférence de 5 à 40 % en poids du poids total de la composition.

16. Procédé de traitement cosmétique pour la protection, le soin, le nettoyage et/ou le maquillage de la peau et/ou des muqueuses et/ou des fibres kératiniques, consistant à appliquer sur la peau et/ou les muqueuses et/ou les fibres kératiniques, une composition selon l'une quelconque des revendications précédentes.

17. Procédé de traitement cosmétique pour lutter contre les signes du vieillissement cutané et/ou améliorer l'éclat du teint et/ou lisser la peau du visage et/ou du corps et/ou traiter les rides et les ridules de la peau et/ou stimuler le processus de renouvellement épidermique, consistant à appliquer sur la peau une composition selon l'une quelconque des revendications 1 à 15.

18. Utilisation de la composition selon l'une quelconque des revendications 1 à 15, pour la fabrication d'une composition dermatologique destinée à lutter contre les signes du vieillissement cutané et/ou à lutter contre l'acné et/ou à lutter contre les désordres cutanés.

19. Procédé pour introduire un dérivé d'acide salicylique de formule (I) ou un sel monovalent ou divalent d'un tel dérivé dans une composition cosmétique et/ou dermatologique contenant de l'eau, consistant à ajouter au dit dérivé au moins un éther d'alcool gras ayant de 12 à 22 atomes de carbone, comprenant au moins un groupement oxyéthyléné et au moins un groupement oxypropyléné.
